# EUROPEAN PATENT APPLICATION

(11) **EP 4 702 980 A1**
(43) Date of publication of application: **04.03.2026**
(21) Application number: 24196408.9
(22) Date of filing: 26.08.2024
(51) Int. Cl.: A61K 31/575, A61P 11/00

(54) **ISOLITHOCHOLIC ACID FOR USE IN THE PREVENTION OR TREATMENT OF INFLAMMATORY DISEASES OF THE RESPIRATORY SYSTEM**

(71) Applicant: Instytut Biologii Doswiadczalnej PAN. im. M. Nenckiego, 02-093 Warszawa (PL)
(72) Inventor: Wypych, Tomasz, 00-740 Warszawa (PL); Bulanda, Edyta, 00-158 Warszawa (PL); Rodriguez-Viso, Pilar, 02-019 Warszawa (PL); Wolska, Magdalena, 02-120 Warszawa (PL)
(74) Representative: JWP Patent & Trademark Attorneys

(57) **Abstract**

The subject matter of the invention is an isolitocholic acid for use in the prevention and/or treatment of inflammatory diseases of the respiratory system and/or prevention and/or treatment of cytokine storm-associated diseases.

## Description

### Field of the invention

The present invention relates to prevention or treatment of inflammatory diseases of the respiratory system and/or prevention or treatment of cytokine storm-associated diseases.

### Background

European patent EP3668879B1 discloses steroid compounds that have a formula represented by the following: (I) and wherein R1, R2, R3a, R3b, R4a, R4b, R5, R6a, R6b, R7, R8, and n are as described herein. The compounds may be prepared as pharmaceutical compositions, and may be used for promoting differentiation of T regulatory (Treg) lymphocytes, and for the prevention and treatment of a variety of conditions in mammals including humans, including by way of non-limiting example, inflammatory conditions, autoimmune disorders, and graft-versus-host disease.

International publication WO2020260558A1 discloses isolithocholic acid (3β-hydroxy-5β-cholan-24-oic acid) and isoallolithocholic acid (3β-hydroxy-5α-cholan-24-oic acid) together with the respective 22-homo-analogs or the deuterated analogs, which are modified in 3-position, for preventing or treating Clostridioides difficile-associated disease in a mammalian subject.

European patent application EP3004132A1 discloses compositions and methods for treating diseases or disorders associated with the metabolic syndrome using conjugates of bile acids with basic amino acids or a decarboxylated amino acid such as agmatine are provided. Further provided are bile acid- basic amino acid conjugates comprising chenodeoxycholic acid with an amino acid selected from arginine, lysine, histidine, ornithine or a decarboxylated amino acid such as agmatine.

Secondary bile acids are microbial products converted from primary bile acids. They have been shown to exert anti-inflammatory properties in the intestines through nuclear receptors and/or G protein-coupled receptors [1, 2]. Several bile acids were shown to be potent modulators of adaptive immunity. Lithocholic acid (LCA) inhibited the activation of Th1 cells in vitro via Vitamin D Receptor (VDR) signalling [3]. 3-oxo-lithocholic acid and isolithocholic acids inhibited Th17 differentiation in vitro and in vivo by binding RORyt, a master regulator of the Th17 subset, and inhibiting its transcriptional activity [4, 5]. Finally, isoallolithocholic acid (isoalloLCA) promoted the generation of mitochondrial reactive oxygen species, leading to enhanced expression of FOXP3 and differentiation of regulatory T cells in vitro [4, 6]. However, the potential impact of secondary bile acids on respiratory immunity has not been described. It is widely accepted that the gut microbiota profoundly shapes our immunity [7-9]. Dysbiosis in patients suffering from conditions characterized by airway inflammation has been described [10-14], which triggered interest in reducing symptoms of these diseases via probiotic administration [15, 16]. However, this approach raises three major concerns. First, probiotics fail to colonize the host, which prevents the long-lasting effect [17]. Second, as live organisms, probiotics adjust their metabolic functions according to new environments they are introduced to (different in terms of the microbiota/immune-metabolic states in each person), and thus, may lose their beneficial potential [18]. Finally, the concentrations of microbial products become diluted along the transit from the gut to the lungs (as demonstrated in the case of short-chain fatty acids [19, 20]), which might hinder their protective effect.

A recent SARS-Cov-2 pandemic illustrated an emerging medical need to develop therapeutic interventions to reduce airway pathology in patients experiencing airway inflammation. One strategy to achieve this outcome is the use of anti-inflammatory drugs. Despite extensive efforts, many clinical trials failed to prove the efficacy of candidate compounds. These include immunomodulators such as anti-interleukin-6 or anti-interleukin-1 monoclonal antibodies [21], hydrocortisone [22], azithromycin [23], doxycycline [24], interferon beta-1a [25], or the Janus kinase (JAK)1/JAK2 inhibitor ruxolitinib [26]. Several therapeutics did show a clinical benefit. These include dexamethasone [27], anti-IL-6R monoclonal antibody Tocilizumab [28], or Janus-associated tyrosine kinase (JAK) 1 and JAK 2 inhibitor, Baricitinib [29]. However, their efficacy is still limited (e.g. the improved mortality rate in the case of dexamethasone treatment is 22.9% vs 25.7% in the control group) [27]. In addition, those drugs are delivered orally or intravenously, which increases the likelihood of off-target effects. The primary mode of action behind these compounds relied on the effective inhibition of neutrophil influx, which is a hallmark of several respiratory diseases, including bacterial or viral infection-induced acute respiratory distress syndrome (ARDS) [30], neutrophilic severe asthma [31], chronic obstructive pulmonary disease (COPD) [32], bronchiectasis [33], or idiopathic pulmonary fibrosis (IPF) [34]. Of note, all of these mediators CXCL1, CCL2, CCL5, CXCL10, and IL-6 have been implicated in the pathogenesis of ARDS, neutrophilic asthma, COPD or IPF [31, 35-43]. Influx of neutrophils into the lung tissue is responsible for immunopathology in ARDS [30, 44, 45]. Moreover, major cause of the immunopathology of ARDS are carried by influx of dendritic cells and pro-inflammatory (Ly6C+) monocytes [46-50]. It is worth to mention that Cystatin C and clusterin, among other markers, have been recommended for use in preclinical development by the Predictive Safety Testing Consortium in collaboration with the US Food and Drug Administration (FDA), the European Medicines Agency and the Japanese Pharmaceuticals and Medical Devices Agency (PMDA) and the number of pharmaceutical companies [52].

Bile acids are known to be recognized by two classes of receptors, nuclear receptors (farnesoid X receptor, pregnane X receptor, vitamin D receptor, RAR-related orphan receptor gamma, the liver X receptor, and Nuclear Receptor Subfamily 4 Group A Member 1 and constitutive androstane receptor) and G protein-coupled receptors (sphingosine-1-phosphate receptor 2, Takeda G protein-coupled receptor 5, and muscarinic acetylcholine receptor M3) [53, 54]. Activation of a nuclear receptor, farnesoid X receptor (FXR) was shown to induce expression of a transcriptional repressor, SHP. In turn, SHP was demonstrated to interact with a transcription factor AP1, preventing AP1-driven expression of inflammatory genes [55], or directly bind to regulatory elements of inflammatory genes [56]. Activation of a G protein-coupled receptor, Takeda G protein-coupled receptor 5 (TGR5, also known as the G protein-coupled bile acid receptor 1, GPBAR1), was reported to activate protein kinase A (PKA) and cAMP-responsive element-binding protein (CREB), thereby reducing NF-κB activity in a STAT-1-dependent fashion [57-59]. Finally, both FXR and TGR5 were demonstrated to inhibit NLRP3 inflammasome activation. TGR5 signaling led to PKA-dependent phosphorylation of NLRP3, which resulted in NLRP3 ubiquitination and prevention of inflammasome assembly. FXR, on the other hand, physically interacted with caspase 1 and NLRP3, which prevented inflammasome assembly [53].

In light of the above, the approach to administer a microbial metabolite or its derivatives directly to the site of the interest overcomes problems with probiotic regimens in the following ways: i) administration of purified microbial compounds does not rely on microbial colonization; ii) the function of administered compounds is not a subject of adaptation to a new environment; iii) their safety is easier to ensure, and iv) the route along the gut-lung axis is bypassed via direct intranasal administration, maximizing bioavailability of the compound in the airways.

### Summary of the invention

The first aspect of the invention is an isolitocholic acid of the following formula: 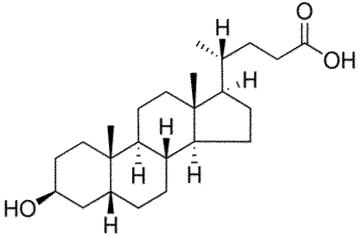 for use in the prevention and/or treatment of inflammatory diseases of the respiratory system and/or prevention and/or treatment of cytokine storm-associated diseases.

Preferably, the prevention and/or treatment of inflammatory diseases of the respiratory system by effective inhibition of neutrophil influx.

Preferably, the prevention and/or treatment of inflammatory diseases of the respiratory system is based on inhibition of the production of one or more of the following cytokine: CXCL1, CCL2, CCL5, CXCL10, IL-6.

Preferably, the inflammatory disease of the respiratory system belongs to a group of such instances as: acute respiratory distress syndrome (ARDS), viral respiratory infections, bacterial respiratory infections, neutrophilic asthma, chronic obstructive pulmonary disease, bronchiectasis, idiopathic pulmonary fibrosis or combination thereof.

The particular advantage of our invention is a localized delivery in a form of a nasal spray and the capacity to inhibit a broad spectrum of inflammatory genes, which increases the efficacy of the treatment. We propose isolithocholic acid (isoLCA) to be formulated as prophylactic and/or therapeutic formulations against respiratory conditions characterized by neutrophilia (including but not limited to: ARDS, viral or bacterial respiratory infections, neutrophilic asthma, chronic obstructive pulmonary disease, bronchiectasis or idiopathic pulmonary fibrosis).

### Brief description of the drawings

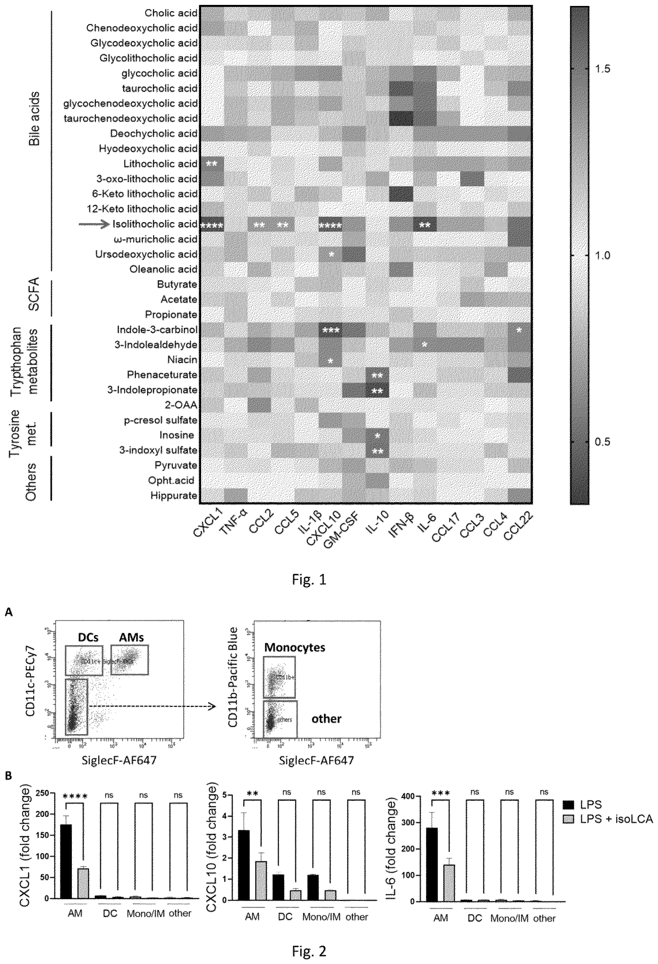

Fig. 1 presents isoLCA suppresses the production of pro-inflammatory mediators; Lung CD45⁺ cells were stimulated with LPS for 24 hours in the presence of metabolites (50 µM, established as non-toxic), and the analytes were measured by cytometric bead arrays. Cells stimulated only with LPS constituted a reference to which a fold change was calculated.*p<0.05, **p<0.01, ***p<0.001, ****p<0.0001
Fig. 2 presents alveolar macrophages (AM) are the target cell type for isoLCA; A) AM (CD11c⁺ SiglecF⁺), DCs (CD11c⁺ SiglecF), monocytes/interstitial macrophages (mono/IM) (CD11c⁻SiglecF⁻ CD11b⁺), and the remaining population (CD11c⁻ SiglecF⁻ CD11b⁻) were sorted with the use of Fluorescence-activated cell sorting and stimulated as per Fig. 1; B) Fold change was calculated relative to unstimulated conditions. Data points represent biological replicates pooled from 4 independent experiments. Error bars represent the SEM. **p<0.01, ***p<0.001, ****p<0.0001

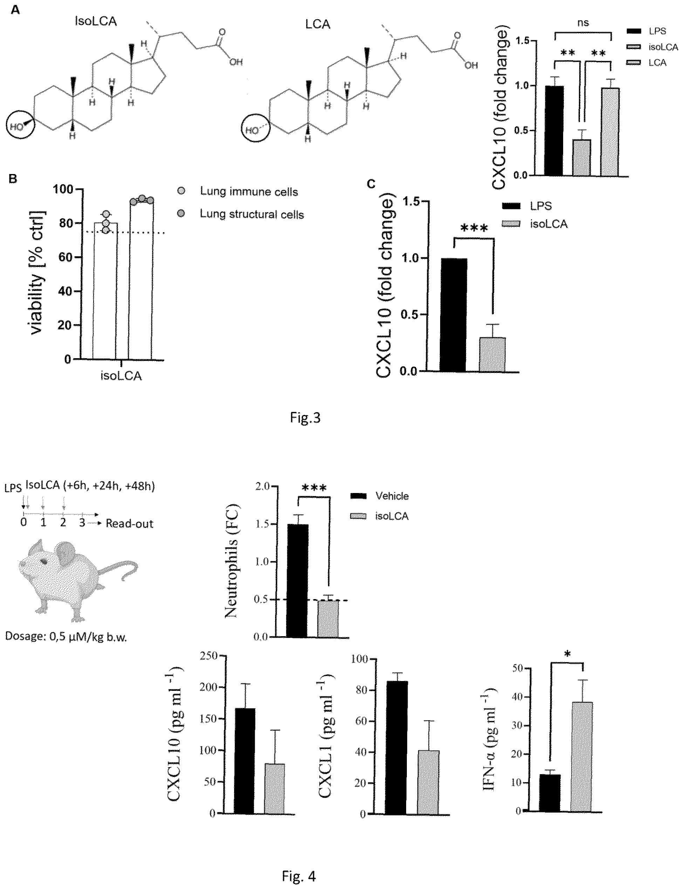

Fig. 3 presents alveolar macrophages (AM) are the target cell type for isoLCA; A) AM (CD11c⁺ SiglecF⁺), DCs (CD11c⁺ SiglecF), monocytes/interstitial macrophages (mono/IM) (CD11c⁻SiglecF⁻ CD11b⁺), and the remaining population (CD11c⁻ SiglecF⁻ CD11b⁻) were sorted with the use of Fluorescence-activated cell sorting and stimulated as per Fig. 1; B) Fold change was calculated relative to unstimulated conditions. Data points represent biological replicates pooled from 4 independent experiments. Error bars represent the SEM. **p<0.01, ***p<0.001, ****p<0.0001
Fig. 4 presents isoLCA as a more effective than its natural isomer isoLCA. A) Activity of LCA, bearing the opposite orientation of the C3-hydroxyl group (at the concentration of 50 µM) B) Cellular toxicity assay of isoLCA at 50 µM in lung immune cells and lung structural cells., C) Activity of isoLCA at 50 µM. Data points represent biological replicates pooled from 4 (A) or 3 (B, C) independent experiments. Error bars represent the SEM. *p<0.05, **p<0.01.

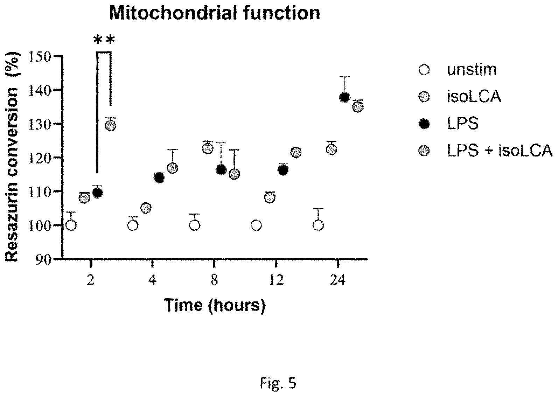

Fig. 5 presents the therapeutic potential of isoLCA in a mouse model of ARDS; Experimental setup. C57BL/6J mice were administered with 20 µg of LPS intranasally. isoLCAwas administered intranasally 6 hours, 24 hours, and 48 hours later at the dose of 0,5 µM/kg body weight. Lungs were collected on day 3; Number of neutrophils was obtained with the use of flow cytometry and calculated as fold change in comparison to the control group (administration of LPS followed by the vehicle treatment); The level of pro-inflammatory cytokines (CXCL10 and CXCL1) decreased, while anti-inflammatory cytokine (IFN-α) was boosted by isoLCA. Data points represent 8 biological replicates pooled from 2 independent experiments. Error bars represent the SEM. *p<0.05, **p<0.01, ****p<0.0001.
Fig. 6 presents the results of isolithocholic acid, which boosts mitochondrial function in inflammatory settings.

### Embodiments

### Embodiment 1 - isoLCA inhibits the pro-inflammatory response in the lungs

We screened the library of metabolites for their capacity to inhibit a panel of inflammatory mediators that are typically observed during acute respiratory distress syndrome. For this, we used lungs isolated from naive mice C57BL/6J bred in the animal house at our Institute, and after using MACS sorting (Miltenyi Biotec), we obtained CD45 positive cells. We stimulated them with LPS (1 µg/ml), which mimics the inflammation in the presence or absence of metabolites in 50 µM concentration, established as non-toxic. After 24h, we collected the supernatant, and the production of inflammatory mediators was analysed by cytometric bead arrays (Legend Plex) or ELISA (R&D or Invitrogen). By stimulating lung immune cells with lipopolysaccharide (a compound that mimics a bacterial infection), we found isoLCA to be the most effective among tested compounds at inhibiting the proinflammatory response. It inhibits the production of CXCL1, CCL2, CCL5, CXCL10 and IL-6 to a statistically significant degree (fold change <0,5 versus LPS alone) (Fig. 1).

### Embodiment 2 - isoLCA acts on alveolar macrophages

Next, we identified a cellular target of isoLCA. For this, using BD FACSAria II cell sorter (BD Bioscience),we sorted major immune cell types from the lungs. Cells were incubated with antibodies of specific fluorochromes and the flow cytometer sorted the cells into different groups based on their fluorescence intensity. Cells were sorted into groups according to their fluorescence intensity and size, in this case into 4 populations: alveolar macrophages ("AMs"), dendritic cells ("DCs"), monocytes, and the remaining population ("other") (Fig. 3A). All populations were stimulated with lipopolysaccharide in the presence/absence of isoLCA. These experiments showed that proinflammatory response was mainly inhibited in alveolar macrophages by isolitocholic acid. That indicates that during inflammation in the lungs, isolitocholic acid affects AMs, leading to a decrease in the production of proinflammatory cytokines by those cells. Therefore, we identified alveolar macrophages as cellular targets of isoLCA (Fig. 2A, 2B).

### Embodiment 3 - isoLCA retain immunosuppressive activity

To target isoLCA for modifications, we first identified the active group of the molecule. We noted that its natural isomer, lithocholic acid (LCA), which differs in the three-dimensional orientation of the C3-hydroxyl group, lost the immunosuppressive potential (Fig. 3A). This data indicated that the C3-hydroxyl group is very important for the immunosuppressive activity of isoLCA involving the inhibition of production proinflammatory cytokines by immune lungs cells and suggested that its modification can alter the magnitude of the effect.

### Embodiment 4 - isoLCA protects mice against ARDS

To test whether identified compounds hold the potential to be developed as drugs against ARDS, we validated our findings in a mouse model of ARDS. C57BL/6J mice bred in the animal house at our Institute were administered with lipopolysaccharide from Escherichia coli O127:B8 (SigmaAldrich) intranasally (20 µg), and isoLCA (Cayman) was administered intranasally periodically 6 hours, 24 hours, and 48 hours later at a dose of 0.5 µM/kg body weight (Fig. 5). After 3 days, mice were sacrificed, and cell infiltration in the lung and cytokine levels in BALF fluid were measured.

IsoLCA potently inhibited the influx of neutrophils in the lung tissue. It also led to a decrease in CXCL1 or CXCL10, consistent with our in vitro data. Finally, isoLCA increased the production of IFN alpha (not observed in vitro). The early boost in IFN-a response is associated with good outcomes of ARDS. So it looks like we are dealing here with an interesting mode of action behind isoLCA: on the one hand, it is able to suppress the pathological cytokine storm; on the other, it can boost a protective response (Fig. 4).

### Embodiment 5 - Mechanism of action

In our laboratory, we have indicated an additional, previously undescribed mechanism whereby isolithocholic acid boosts mitochondrial function in inflammatory settings. We observed that isolithocholic acid is capable of boosting mitochondria activity by increasing resazurin conversion and protecting the loss of membrane potential induced by LPS. Alveolar macrophages showed reduced capacity to produce proinflammatory cytokines and chemokines, as well as reactive oxygen species. Therefore, we established that isolithocholic acid leads to metabolic modulation of alveolar macrophages (Fig. 5).

### References:

1. Wahlstrom, A., et al., Intestinal Crosstalk between Bile Acids and Microbiota and Its Impact on Host Metabolism. Cell Metab, 2016. 24(1): p. 41-50.
2. Fiorucci, S. and E. Distrutti, Bile Acid-Activated Receptors, Intestinal Microbiota, and the Treatment of Metabolic Disorders. Trends Mol Med, 2015. 21(11): p. 702-714.
3. Pols, T.W.H., et al., Lithocholic acid controls adaptive immune responses by inhibition ofTh1 activation through the Vitamin D receptor. PLoS One, 2017. 12(5): p. e0176715.
4. Hang, S., et al., Bile acid metabolites control TH17 and Treg cell differentiation. Nature, 2019. 576(7785): p. 143-148.
5. Paik, D., et al., Human gut bacteria produce TauEta17-modulating bile acid metabolites. Nature, 2022. 603(7903): p. 907-912.
6. Tanoue, T., et al., A defined commensal consortium elicits CD8 T cells and anti-cancer immunity. Nature, 2019. 565(7741): p. 600-605.
7. Ivanov, II and K. Honda, Intestinal commensal microbes as immune modulators. Cell Host Microbe, 2012. 12(4): p. 496-508.
8. Wypych, T.P. and B.J. Marsland, Antibiotics as Instigators of Microbial Dysbiosis: Implications for Asthma and Allergy. Trends Immunol, 2018. 39(9): p. 697-711.
9. Wypych, T.P., L.C. Wickramasinghe, and B.J. Marsland, The influence of the microbiome on respiratory health. Nat Immunol, 2019. 20(10): p. 1279-1290.
10. Zuo, T., et al., Alterations in Gut Microbiota of Patients With COVID-19 During Time of Hospitalization. Gastroenterology, 2020. 159(3): p. 944-955 e8.
11. Yeoh, Y.K., et al., Gut microbiota composition reflects disease severity and dysfunctional immune responses in patients with COVID-19. Gut, 2021. 70(4): p. 698-706.
12. Barcik, W., et al., The Role of Lung and Gut Microbiota in the Pathology of Asthma. Immunity, 2020. 52(2): p. 241-255.
13. Hufnagl, K., et al., Dysbiosis of the gut and lung microbiome has a role in asthma. Semin Immunopathol, 2020. 42(1): p. 75-93.
14. Li, N., et al., Gut microbiota dysbiosis contributes to the development of chronic obstructive pulmonary disease. Respir Res, 2021. 22(1): p. 274.
15. Hussain, I., et al., Role of Gut Microbiome in COVID-19: An Insight Into Pathogenesis and Therapeutic Potential. Front Immunol, 2021. 12: p. 765965.
16. Mortaz, E., et al., Probiotics in the management of lung diseases. Mediators Inflamm, 2013. 2013: p. 751068.
17. Zmora, N., et al., Personalized Gut Mucosal Colonization Resistance to Empiric Probiotics Is Associated with Unique Host and Microbiome Features. Cell, 2018. 174(6): p. 1388-1405 e21.
18. Suez, J., et al., The pros, cons, and many unknowns of probiotics. Nat Med, 2019. 25(5): p. 716-729.
19. Yue, M., et al., Measurement of Short-Chain Fatty Acids in Respiratory Samples: Keep Your Assay above the Water Line. Am J Respir Crit Care Med, 2020. 202(4): p. 610-612.
20. Cummings, J.H., et al., Short chain fatty acids in human large intestine, portal, hepatic and venous blood. Gut, 1987. 28(10): p. 1221-7.
21. Declercq, J., et al., Effect of anti-interleukin drugs in patients with COVID-19 and signs of cytokine release syndrome (COV-AID): a factorial, randomised, controlled trial. Lancet Respir Med, 2021. 9(12): p. 1427-1438.
22. Dequin, P.F., et al., Effect of Hydrocortisone on 21-Day Mortality or Respiratory Support Among Critically III Patients With COVID-19: A Randomized Clinical Trial. JAMA, 2020. 324(13): p. 1298-1306.
23. Oldenburg, C.E., et al., Effect of Oral Azithromycin vs Placebo on COVID-19 Symptoms in Outpatients With SARS-CoV-2 Infection: A Randomized Clinical Trial. JAMA, 2021. 326(6): p. 490-498.
24. Butler, C.C., et al., Doxycycline for community treatment of suspected COVID-19 in people at high risk of adverse outcomes in the UK (PRINCIPLE): a randomised, controlled, open-label, adaptive platform trial. Lancet Respir Med, 2021. 9(9): p. 1010-1020.
25. Kalil, A.C., et al., Efficacy of interferon beta-1a plus remdesivir compared with remdesivir alone in hospitalised adults with COVID-19: a double-bind, randomised, placebo-controlled, phase 3 trial. Lancet Respir Med, 2021. 9(12): p. 1365-1376.
26. Han, M.K., et al., Ruxolitinib in addition to standard of care for the treatment of patients admitted to hospital with COVID-19 (RUXCOVID): a randomised, double-blind, placebo-controlled, phase 3 trial. Lancet Rheumatol, 2022. 4(5): p. e351-e361.
27. Group, R.C., et al., Dexamethasone in Hospitalized Patients with Covid-19. N Engl J Med, 2021. 384(8): p. 693-704.
28. Writing Committee for the, R.-C.A.P.I., et al., Long-term (180-Day) Outcomes in Critically III Patients With COVID-19 in the REMAP-CAP Randomized Clinical Trial. JAMA, 2023. 329(1): p. 39-51.
29. Marconi, V.C., et al., Efficacy and safety of baricitinib for the treatment of hospitalised adults with COVID-19 (COV-BARRIER): a randomised, double-blind, parallel-group, placebo-controlled phase 3 trial. Lancet Respir Med, 2021. 9(12): p. 1407-1418.
30. Yang, S.C., et al., Understanding the role of neutrophils in acute respiratory distress syndrome. Biomed J, 2021. 44(4): p. 439-446.
31. Zhang, X., et al., The onset, development and pathogenesis of severe neutrophilic asthma. Immunol Cell Biol, 2022. 100(3): p. 144-159.
32. Hoenderdos, K. and A. Condliffe, The neutrophil in chronic obstructive pulmonary disease. Am J Respir Cell Mol Biol, 2013. 48(5): p. 531-9.
33. Summers, C., Chasing the "Holy Grail": Modulating Neutrophils in Inflammatory Lung Disease. Am J Respir Crit Care Med, 2019. 200(2): p. 131-132.
34. Mincham, K.T., et al., Our evolving view of neutrophils in defining the pathology of chronic lung disease. Immunology, 2021. 164(4): p. 701-721.
35. Chu, H., et al., Comparative Replication and Immune Activation Profiles of SARS-CoV-2 and SARS-CoV in Human Lungs: An Ex Vivo Study With Implications for the Pathogenesis of COVID-19. Clin Infect Dis, 2020. 71(6): p. 1400-1409.
36. Del Valle, D.M., et al., An inflammatory cytokine signature predicts COVID-19 severity and survival. Nat Med, 2020. 26(10): p. 1636-1643.
37. Zaid, Y., et al., Chemokines and eicosanoids fuel the hyperinflammation within the lungs of patients with severe COVID-19. J Allergy Clin Immunol, 2021. 148(2): p. 368-380 e3.
38. Harrison, C., Focus shifts to antibody cocktails for COVID-19 cytokine storm. Nat Biotechnol, 2020. 38(8): p. 905-908.
39. Santa Cruz, A., et al., Interleukin-6 Is a Biomarker for the Development of Fatal Severe Acute Respiratory Syndrome Coronavirus 2 Pneumonia. Front Immunol, 2021. 12: p. 613422.
40. Lai, C., et al., C-C Motif Chemokine Ligand 2 (CCL2) Mediates Acute Lung Injury Induced by Lethal Influenza H7N9 Virus. Front Microbiol, 2017. 8: p. 587.
41. Williams, A.E., et al., Evidence for chemokine synergy during neutrophil migration in ARDS. Thorax, 2017. 72(1): p. 66-73.
42. Chung, K.F. and I.M. Adcock, Multifaceted mechanisms in COPD: inflammation, immunity, and tissue repair and destruction. Eur Respir J, 2008. 31(6): p. 1334-56.
43. Schupp, J.C., et al., Macrophage activation in acute exacerbation of idiopathic pulmonary fibrosis. PLoS One, 2015. 10(1): p. e0116775.
44. Grommes, J. and O. Soehnlein, Contribution of neutrophils to acute lung injury. Mol Med, 2011. 17(3-4): p. 293-307.
45. Zemans, R.L. and M.A. Matthay, What drives neutrophils to the alveoli in ARDS? Thorax, 2017. 72(1): p. 1-3.
46. Dhaliwal, K., et al., Monocytes control second-phase neutrophil emigration in established lipopolysaccharide-induced murine lung injury. Am J Respir Crit Care Med, 2012. 186(6): p. 514-24.
47. Maus, U., et al., The role of CC chemokine receptor 2 in alveolar monocyte and neutrophil immigration in intact mice. Am J Respir Crit Care Med, 2002. 166(3): p. 268-73.
48. Mo, J., et al., Single-cell analysis reveals dysregulated inflammatory response in peripheral blood immunity in patients with acute respiratory distress syndrome. Front Cell Dev Biol, 2023. 11: p. 1199122.
49. Li, X.C., M. Miyasaka, and T.B. Issekutz, Blood monocyte migration to acute lung inflammation involves both CD11/CD18 and very late activation antigen-4-dependent and independent pathways. J Immunol, 1998. 161(11): p. 6258-64.
50. Li, L., et al., Classical dendritic cells regulate acute lung inflammation and injury in mice with lipopolysaccharide-induced acute respiratory distress syndrome. Int J Mol Med, 2019. 44(2): p. 617-629.
51. Thomas, G., et al., Nonclassical patrolling monocyte function in the vasculature. Arterioscler Thromb Vasc Biol, 2015. 35(6): p. 1306-16.
52. Campion, S., et al., The current status of biomarkers for predicting toxicity. Expert Opin Drug Metab Toxicol, 2013. 9(11): p. 1391-408.
53. Fiorucci, S., et al., Bile Acids Activated Receptors Regulate Innate Immunity. Front Immunol, 2018. 9: p. 1853.
54. Godlewska, U., E. Bulanda, and T.P. Wypych, Bile acids in immunity: Bidirectional mediators between the host and the microbiota. Front Immunol, 2022. 13: p. 949033.
55. Fiorucci, S., et al., The nuclear receptor SHP mediates inhibition of hepatic stellate cells by FXR and protects against liver fibrosis. Gastroenterology, 2004. 127(5): p. 1497-512.
56. Yang, Z., A.N. Koehler, and L. Wang, A Novel Small Molecule Activator of Nuclear Receptor SHP Inhibits HCC Cell Migration via Suppressing Ccl2. Mol Cancer Ther, 2016. 15(10): p. 2294-2301.
57. Haselow, K., et al., Bile acids PKA-dependently induce a switch of the IL-10/IL-12 ratio and reduce proinflammatory capability of human macrophages. J Leukoc Biol, 2013. 94(6): p. 1253-64.
58. Biagioli, M., et al., The Bile Acid Receptor GPBAR1 Regulates the M1/M2 Phenotype of Intestinal Macrophages and Activation of GPBAR1 Rescues Mice from Murine Colitis. J Immunol, 2017. 199(2): p. 718-733.
59. Perino, A., et al., TGR5 reduces macrophage migration through mTOR-induced C/EBPbeta differential translation. J Clin Invest, 2014. 124(12): p. 5424-36.

## Claims

1. Isolitocholic acid of the following formula: 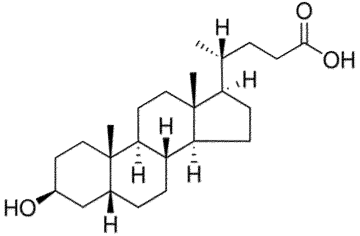 for use in the prevention and/or treatment of inflammatory diseases of the respiratory system and/or prevention and/or treatment of cytokine storm-associated diseases.

2. A compound for use according to claim 1, **characterised in that**, the prevention and/or treatment of inflammatory diseases of the respiratory system by effective inhibition of neutrophil influx.

3. A compound for use according to any one of claims 1-2, **characterised in that**, the prevention and/or treatment of inflammatory diseases of the respiratory system is based on inhibition of the production of one or more of the following cytokine: CXCL1, CCL2, CCL5, CXCL10, IL-6.

4. A compound for use according to any one of claim 1-3, **characterised in that**, the inflammatory disease of the respiratory system belongs to a group of such instances as: ARDS, viral respiratory infections, bacterial respiratory infections, neutrophilic asthma, chronic obstructive pulmonary disease, bronchiectasis, idiopathic pulmonary fibrosis or combination thereof.
